# EUROPEAN PATENT APPLICATION

(11) **EP 2 557 179 A1**
(43) Date of publication of application: **13.02.2013**
(21) Application number: 11766005.0
(22) Date of filing: 08.04.2011
(51) Int. Cl.: C12Q 1/68, C12N 15/09, G01N 33/50

(54) **TEST METHOD FOR DETERMINING SUSCEPTIBILITY TO OR STATE OF IMMUNE SYSTEM DISORDER OR JOINT DISORDER**

(30) Priority: 08.04.2010 JP 2010101467
(71) Applicant: Yoshitomi, Hiroyuki, Kyoto-shi, Kyoto 606-8507 (JP); Mitsubishi Chemical Medience Corporation, Tokyo 108-8559 (JP)
(72) Inventor: YOSHITOMI, Hiroyuki, Kyoto-shi, Kyoto 606-8507 (JP); MURATA, Koichi, Kyoto-shi, Kyoto 606-8507 (JP)
(74) Representative: Vossius & Partner
(86) International application number: PCT/JP2011/058875
(87) International publication number: WO 2011/126105

(57) **Abstract**

The present invention provides an examination method for determining the contraction or the activity of diseases related to immune system and/or joint system, comprising measuring the expression level of miRNAs in a blood-derived or joint-derived fluid sample. The present invention is an examination method for determining the activity of diseases related to immune system and/or joint system, comprising: preparing blood-derived fluid samples collected over time, measuring the expression levels of at least an miRNA selected from SEQ ID NOS: 1. to 5 in the fluid samples, and comparing the expression levels between different sampling times.

## Description

### TECHNICAL FIELD

The present invention relates to an examination method for determining the contraction or the activity of diseases related immune system and/or joint system, by measuring the expression level of miRNA in a blood-derived or joint-derived fluid sample.

### BACKGROUND ART

MicroRNAs (hereinafter referred to as miRNAs) are small (approximately 22 nucleotides) noncoding RNAs (non-patent literature 1). miRNAs regulate the translation of target mRNAs by binding at sites in the 3'untranslated region (3'UTR) of the mRNAs, and are implicated in various processes such as cell metabolism, organ formation, and malignant tumors.
It had been considered that RNAs are not present in sera or plasma from which cellular components are removed, because RNases are present in blood, and an RNA added is immediately decomposed. However, it was recently shown that miRNA-141 is a specific biomarker for bladder cancer in sera or plasma (non-patent literature 2).
It is known that intracellular miRNAs do not generally correspond to miRNAs released from cells (non-patent literature 3). Therefore, the variations in sera or plasma of miRNAs, which are increased or decreased in cells or tissues, are uncertain and unpredictable.

### CITATION LIST

### NON-PATENT LITERATURE

[Non-patent literature 1] Ambros V: microRNAs: tiny regulators with great potential. Cell 2001, 107: 823-826.
[Non-patent literature 21 Mitchell PS, et al. Circulating microRNAs as stable blood-based markers for cancer detection. Proc Natl Acad Sci U S A 2008, 105: 10513-10518.
[Non-patent literature 3] Valadi H, et al. Exosome-mediated transfer of mRNAs and microRNAs is a novel mechanism of genetic exchange between cells. Nat Cell Biol 2007, 9: 654-659.

### SUMMARY OF INVENTION

### TECHNICAL PROBLEM

An object of the present invention is to provide a method for detecting the contraction of diseases related immune system and/or joint system (an in vitro diagnostic method), and to provide an examination method in which a sample collection and control is easy and diseases can be examined with high accuracy.

### SOLUTION TO PROBLEM

The inventors of the present invention have conducted intensive studies to solve the object, and have found (1) that the contraction of diseases related immune system and/or joint system can be determined using specific miRNAs contained in a blood-derived fluid sample as a biomarker, (2) that specific miRNAs are present in a joint-derived fluid sample, and diseases related immune system and/or joint system can be differentiated using these miRNAs as a biomarker, (3) that the activity of diseases related immune system and/or joint system can be examined using specific miRNAs contained in a blood-derived fluid sample, and in particular, (4) that the activity of diseases related immune system and/or joint system can be examined using a ratio obtained by dividing the concentration of one of specific miRNAs in a joint-derived fluid sample by the concentration of the miRNA in a blood-derived fluid sample, and the present invention has been completed. The present invention includes the following:

[1] A method for detecting diseases related to immune system and/or joint system, wherein at least an miRNA selected from the group consisting of SEQ ID NOS: 1 to 5 contained in a blood-derived or joint-derived fluid sample is used as an index.
[2] An examination method for determining the activity of diseases related to immune system and/or joint system, comprising: preparing blood-derived or joint-derived fluid samples collected over time, measuring the expression levels of at least an miRNA selected from SEQ ID NOS: 1 to 5 in the fluid samples, and comparing the expression levels between different sampling times.
[3] An examination method for determining the activity of diseases related to immune system and/or joint system, comprising: preparing blood-derived fluid samples and joint-derived fluid samples collected over time, measuring the expression levels of at least an miRNA selected from SEQ ID NOS: 1 to 5 in the fluid samples, and comparing ratios (Ea/Eb) of the expression level (Ea) of miRNA obtained from the joint-derived fluid samples to the expression level (Eb) of miRNA obtained from the blood-derived fluid samples between different sampling times.
[4] An examination method for determining the contraction of diseases related to immune system and/or joint system, comprising: measuring the expression level of miRNA of SEQ ID NO: 2 in a blood-derived fluid sample.
[5] An examination method for differentiating diseases related to immune system and/or joint system, comprising: measuring the expression level of at least an miRNA selected from the group consisting of SEQ ID NOS: 1, 3, 4, and 5 in a joint-derived fluid sample.
[6] The method of any one of [1] to [5], wherein the blood-derived fluid sample is plasma, and the joint-derived fluid sample is synovial fluid.
[7] Use of a primer or a probe capable of analyzing the expression level of miRNA selected from the group consisting of SEQ ID NOS: 1 to 5 in the manufacture of a kit for detecting diseases related to immune system and/or joint system.
   The term "fluid sample" as used herein means a liquid prepared by removing cellular components from a sample collected.

### ADVANTAGEOUS EFFECTS OF INVENTION

According to the present invention, even a sample from which cellular components are removed can be examined with high accuracy, and thus, the conventional temperature and time control which was necessary to keep the cell activity can be omitted.
Further, an examination can be carried out without the collection of joint-related tissues such as a synovial tissue or a bone tissue, and thus, the distress in a patient and the possibility of complications can be reduced.

### BRIEF DESCRIPTION OF THE DRAWINGS

Fig. 1 shows the concentrations of miRNAs of SEQ ID NOS: 1 to 5 contained in synovial fluid collected from patients with rheumatoid arthritis (RA) and osteoarthritis (OA). The sample number was 30 for each disease.
Fig. 2 shows ROC curves and areas under the ROC curves (AUCs) for determination of RA patients or OA patients using miRNA of SEQ ID NO: 2 in plasma. The sample number was 30 for each group.
FIG. 3 shows the correlation between plasma expression levels of miRNAs of SEQ ID NOS: 1 to 5 in samples collected from RA patients and TJC or DAS28.
FIG. 4 shows the correlation between synovial fluid miRNA/plasma miRNA ratios and TJC, with respect to miRNAs of SEQ ID NOS: 1 to 5 in samples collected from RA patients.

### DESCRIPTION OF EMBODIMENTS

Hereinafter the present invention will be explained in detail.
The detection (in vitro diagnosis) of diseases related to immune system and/or joint system in the method of the present invention is not limited to, but includes, for example, determination of the contraction of the diseases, differentiation of the diseases, and determination of the activity of the diseases.
In the present invention, the expression level of at least one miRNA selected from miRNAs consisting of the sequences of SEQ ID NOS: 1 to 5, as shown Table 1, in a blood-derived or joint-derived fluid sample is used as an index to detect diseases related to immune system and/or joint system.

**Table 1**

| | | |
|---|---|---|
| **SEQ ID NO: 1** | **hsa-miR-16** | **uagcagcacguaaauauuggcg** |
| **SEO ID NO: 2** | **hsa-miR-132** | **uaacagucuacagccauggucg** |
| **SEQ ID NO: 3** | **hsa-miR-146a** | **ugagaacugaauuccauggguu** |
| **SEQ ID NO: 4** | **hsa-miR-155** | **uuaaugcuaaucgugauaggggu** |
| **SEQ ID NO: 5** | **hsa-miR-223** | **ugucaguuugucaaauacccca** |

Examples of the blood-derived fluid sample include a serum, plasma, and a culture supernatant of blood-derived cells (a supernatant obtained by cultivating blood-derived cells in a culture medium). In particular, a plasma sample is preferred from the viewpoint of convenience. These fluid samples may be collected in a known or conventional method.

Examples of the joint-derived fluid sample include a synovial fluid supernatant, and culture supernatants of various tissue cells associated with joint (for example, articular cartilage, a synovial tissue, a bone tissue, and a ligament tissue, and cells derived from these tissues)(supernatants obtained by cultivating joint-associated cells in a culture medium). In particular, a synovial fluid supernatant is preferred from the viewpoint of convenience. These fluid samples may be collected in a known or conventional method.

Examples of the diseases related to immune system which may be examined by the present invention include rheumatoid arthritis, juvenile rheumatoid arthritis, rheumatic fever, Basedow disease, Hashimoto's disease, systemic lupus erythematosus, periarteritis nodosa, polyarteritis nodosa, mixed connective tissue disease, polymyositis, dermatomyositis, sarcoidosis, scleroderma, psoriatic arthritis, Behcet's disease, seronegative arthritis, ankylosing spondylitis, ulcerative colitis, Crohn's disease, atopic dermatitis, polymyalgia rheumatica, autoimmune hepatitis, relapsing polychondritis, multiple sclerosis, viral infections, sepsis, Sjogren's syndrome, antiphospholipid antibody syndrome, adult Still's disease, and amyloidosis. Rheumatoid arthritis, juvenile rheumatoid arthritis, and rheumatic fever are preferred, and in particular, rheumatoid arthritis can be examined with high accuracy. Since rheumatoid arthritis and juvenile rheumatoid arthritis are accompanied by the destruction of joint, they are also included in the diseases related to joint system.
Examples of the diseases related to joint system which may be examined by the present invention other than these diseases include osteoarthritis, cartilage damage, gout, pseudogout, joint infection (septic arthritis), skeletal dysplasia, fractures, intra-articular ligament injuries, meniscus injury, sprain, bone necrosis, osteoporosis, Charcot's arthritis, cartilage bone tumors, and soft tissue tumors. Osteoarthritis, cartilage damage, fractures, intra-articular ligament injuries, meniscus injury, sprain, and bone necrosis are preferred, and in particular, osteoarthritis can be examined with high accuracy.

The method of the present invention is useful for mammals. Examples of the mammals include a human, anthropoids, rodents, dogs, cats, and rabbits, and a human is preferred.

In the method of the present invention, for example, at first, RNA is extracted from a blood-derived or joint-derived fluid sample taken from a patient. Examples of the RNA extraction include a guanidine/cesium chloride ultracentrifugation method, an acid phenol method, and a spin column method.
Next, miRNA contained in the RNA obtained from the blood-derived or joint-derived fluid sample is detected. This detection method is not limited, so long as the expression level of miRNA can be measured, and examples of the detection method include a real-time RT-PCR method and a microarray method. These methods are described in detail, for example, in Kazuyoshi Kofu, microRNA Jikken Purotokoru (microRNA Experimental Protocols), YODOSHA Co., Ltd.
A primer or a probe capable of analyzing the expression level of miRNA selected from the group consisting of SEQ ID NOS: 1 to 5 can be easily designed, synthesized, and used for those skilled in the art in accordance with their nucleotide sequences.
The kit of the present invention for analyzing the expression level of miRNA comprises at least one primer or probe capable of analyzing the expression level of miRNA selected from the group consisting of SEQ ID NOS: 1 to 5. The kit may optionally comprise a buffer, an enzyme, and the like in accordance with the measurement system.

Hereinafter the present invention will be further explained by embodiments of the present invention.
The present invention includes, as the first embodiment, an examination method for determining the activity of diseases related to immune system and/or joint system, comprising: preparing blood-derived or joint-derived fluid samples collected over time, measuring the expression levels of at least one miRNA selected from SEQ ID NOS: 1 to 5 in the fluid samples, and comparing the expression levels between different sampling times.

In a patient suffering with diseases related to immune system and/or joint system, the expression levels of at least one miRNA selected from SEQ ID NOS: 1 to 5 in the fluid samples may be measured over time, and may be compared with each other between different sampling times. As a result, a significant correlation of the expression levels with the activity of the diseases can be found, and it can be judged, for example, that the activity of the diseases progresses along with a decrease in the expression level of the miRNA. The term "activity of a disease" as used herein means momentum in the progress of the disease, and a decrease over time in the expression level of miRNA indicates that the disease tends to get worse. The interval in the sequential sample collection may be appropriately selected for determining the activity in a patient, and the interval between sampling may be, for example, 7 days to 1 year, and preferably 30 days to 180 days.

The present invention includes, as the second embodiment, an examination method for determining the activity of diseases related to immune system and/or joint system, using at least one miRNA selected from SEQ ID NOS: 1 to 5 (preferably SEQ ID NOS: 1 to 3). This examination method comprises: preparing blood-derived fluid samples and joint-derived fluid samples collected over time, measuring the expression levels of at least one miRNA selected from SEQ ID NOS: 1 to 5 in the fluid samples, and comparing ratios (Ea/Eb) of the expression level (Ea) of miRNA obtained from the joint-derived fluid samples to the expression level (Eb) of miRNA obtained from the blood-derived fluid samples between different sampling times.

In the examination method of the second embodiment, each fluid sample to obtain the ratio (Ea/Eb) for the expression levels may be collected from the same patient, for example, within 7 days, and preferably within 1 day, and the sampling may be carried out over time (for example, at an interval of 7 days to 1 year, and preferably at an interval of 30 days to 180 days). As a result, a significant correlation of the ratios with the activity of the diseases can be found, and it can be judged, for example, that the activity of the diseases progresses along with an increase in the ratio (Ea/Eb).
In the examination method of the second embodiment, the ratio (Ea/Eb) for the expression levels of miRNA is preferably a ratio of an miRNA concentration in synovial fluid to an miRNA concentration in plasma.

The present invention includes, as the third embodiment, an examination method for determining the contraction of diseases related to immune system (for example, rheumatoid arthritis) and/or diseases related to joint system (for example, osteoarthritis), by measuring the expression level of miRNA of SEQ ID NO: 2 in a blood-derived fluid sample collected from a subject.
In this examination method, the expression level of miRNA of SEQ ID NO: 2 collected from a patient may be compared to that of miRNA of SEQ ID NO: 2 collected from a healthy person to determine a threshold in accordance with a statistical method described below, and as a result, a significant correlation for the contraction of diseases related to immune system and/or joint system in the patient is shown.
More particularly, for example, when the concentration of miRNAs of SEQ ID NO: 2 in plasma of a subject is 67 pmol/L or less as a threshold, it can be determined that the subject is suffering with diseases related to immune system (for example, rheumatoid arthritis) and/or diseases related to joint system (for example, osteoarthritis), at approximately 84% of sensitivity and approximately 80% of specificity. In connection of this, this threshold is an example, and the threshold may be appropriately varied in accordance with a statistical analysis.

The present invention includes, as the fourth embodiment, an examination method for differentiating diseases related to immune system (for example, rheumatoid arthritis) and/or diseases related to joint system (for example, osteoarthritis), by measuring the expression level of at least one miRNA selected from the group consisting of SEQ ID NOS: 1, 3, 4, and 5 in a joint-derived fluid sample.
In this examination method, the expression level of the miRNA(s) present in a joint-derived fluid sample collected from a subject may be measured to determine a threshold in accordance with a statistical method described below, and as a result, a significant correlation showing which diseases related to immune system and/or joint system the subject is suffering with is shown.
For example, patients suffering with rheumatoid arthritis show higher levels of miRNA expression than those of osteoarthritis patients. Therefore, miRNAs of SEQ ID NOS: 1, 3, 4, and 5 can be used for differentiation of osteoarthritis at thresholds of 40 pmol/L or less (75% of sensitivity and 78% of specificity), 11 pmol/L or less (73% of sensitivity and 72% of specificity), 0.04 pmol/L or less (77% of sensitivity and 70% of specificity), and 2.5 pmol/L or less (56% of sensitivity and 80% of specificity), respectively. By contrast, when the positive values for rheumatoid arthritis are set at 40 pmol/L or more, 11 pmol/L or more, 0.04 pmol/L or more, and 2.5 pmol/L or more, miRNAs of SEQ ID NOS: 1, 3, 4, and 5 can be used for differentiation of rheumatoid arthritis, at 75% of sensitivity and 78% of specificity, at 73% of sensitivity and 72% of specificity, at 77% of sensitivity and 70% of specificity, and at 56% of sensitivity and 80% of specificity, respectively. In connection of this, these thresholds are examples, and the thresholds may be appropriately varied in accordance with a statistical analysis.

### EXAMPLES

The present invention now will be further illustrated by, but is by no means limited to, the following Examples.

### [Materials and methods]

### (Experimental group)

Ethical approval for this study was granted by the ethics committee of Kyoto University Graduate School and Faculty of Medicine. Peripheral blood and synovial fluid were obtained from 30 patients with rheumatoid arthritis (RA) and 30 patients with osteoarthritis (OA), and peripheral blood was obtained from 30 healthy persons. Peripheral blood samples were collected with tubes containing EDTA-2K.

### (Preparation of fluid samples)

Peripheral blood samples and synovial fluid samples were centrifuged at 400 g for 7 minutes to remove cellular components from the samples, and the resulting supernatants were frozen and stored at -20°C until analysis.

### (Extraction of total RNA)

To 100 µL of plasma or synovial fluid supernatant, 750 µL of Isogen LS (Nippongene), which was a reagent for an acid phenol method, was added to inactivate the RNase activity, and 25 fmol of synthetic cel-miR-39 was added as an external standard to obtain each suspension. After 200 µL of chloroform was added to each suspension, the mixtures were centrifuged at 12,000 g for 15 minutes to collect 300 µL of each aqueous phase. High Pure miRNA Isolation Kit (Roche) was used to extract total. RNA from each aqueous phase.

### (Real-time RT-PCR)

After cDNA was synthesized using NCode VILO miRNA cDNA Synthesis Kit (Invitrogen), real-time RT-PCR was carried out using ABI Prism 7300 Sequence Detection System (Applied Biosystems) and EXPRESS SYBR GreenER qPCR SuperMix (Invitrogen). The primers used were as follows:
hsa-miR-16, 5'-TAG-CAG-CAC-GTA-AAT-ATT-GGC-G-3'(SEQ ID NO: 6)
hsa-miR-132, 5'-TAA-CAG-TCT-ACA-GCC-ATG-GTC-G-3' (SEQ ID NO: 7)
hsa-miR-146a, 5'-TGA-GAA-CTG-AAT-TCC-ATG-GGT-T-3'(SEQ ID NO: 8)
hsa-miR-155, 5'-TTA-ATG-CTA-ATC-GTG-ATA-GGG-GTA-3'(SEQ ID NO: 9)
hsa-miR-223, 5'-TGT-CAG-TTT-GTC-AAA-TAC-CCC-A-3'(SEQ ID NO: 10)
cel-miR-.39, 5'-CGT-CAC-CGG-GTG-TAA-ATC-AGC-TTG-3'(SEQ ID NO: 11)
A primer attached to the NCode VILO miRNA cDNA Synthesis Kit was used as a reverse primer.
To quantify the expression level of each miRNA, each PCR product was previously inserted into pTAC-1 vector (BioDynamics Laboratory), and standard curves were generated using serial dilutions of each of the resulting constructions. The absolute concentration of miRNA contained in each sample was calculated, based on cel-miR-39 which had been added to each sample at a known concentration.

### (Statistical analysis)

(1) miRNA concentrations in synovial fluid from patients with rheumatoid arthritis and patients with osteoarthritis were analyzed with two-tailed t test.
(2) Differences between rheumatoid arthritis and osteoarthritis in miRNA concentrations in plasma from patients with rheumatoid arthritis and patients with osteoarthritis were analyzed with two-tailed t test.
(3) For patients with rheumatoid arthritis, correlations of TJC (tender joint count) or DAS28 score (these indicate the activity of rheumatoid arthritis) with (a) miRNA concentration in plasma, (b) miRNA concentration in synovial fluid, and (c) the ratio (Ea/Eb) obtained by dividing the miRNA concentration in synovial fluid by the miRNA concentration in plasma were statistically analyzed with Pearson product-moment correlation coefficient.

### [Results]

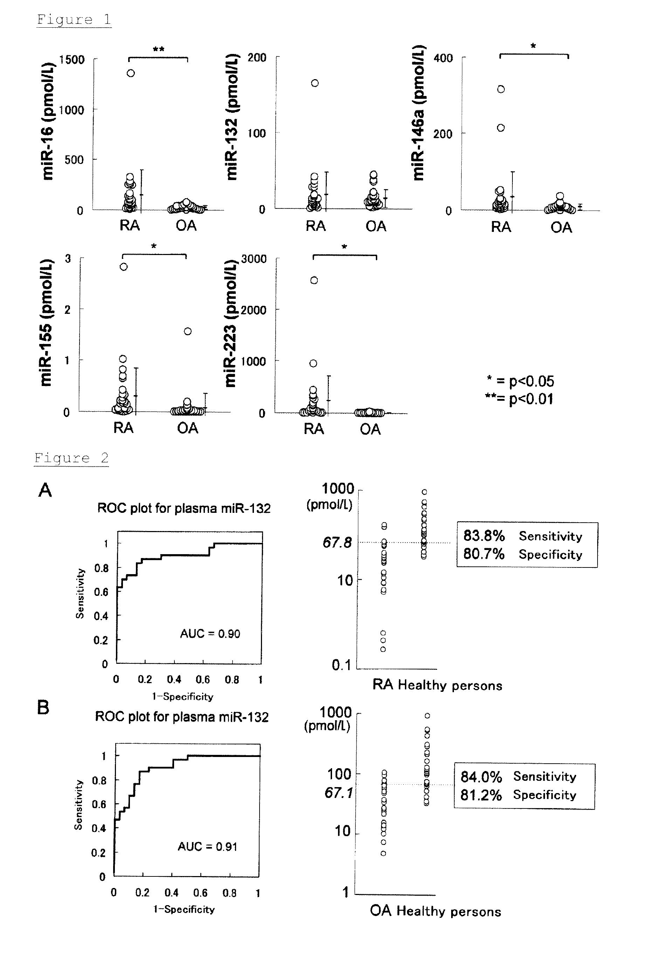

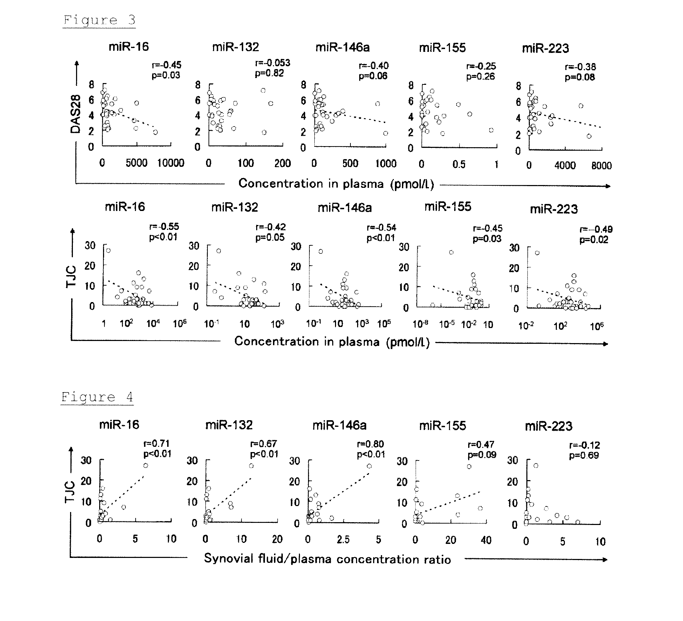

As shown in Figure 1, the concentrations of miR-16, miR-146a, miR-155, and miR-223 in synovial fluid were higher in patients with rheumatoid arthritis than those in patients with osteoarthritis (p<0.05). When the positive values for rheumatoid arthritis are set at 40 pmol/L or more, 11 pmol/L or more, 0.04 pmol/L or more, and 2.5 pmol/L or more, miRNAs of miR-16, miR-146a, miR-155, and miR-223 can be used for differentiation of rheumatoid arthritis, at 75% of sensitivity and 78% of specificity, at 73% of sensitivity and 72% of specificity, at 77% of sensitivity and 70% of specificity, and at 56% of sensitivity and 80% of specificity, respectively.
As shown in Figure 2, the concentration of miR-132 in plasma derived from healthy persons was significantly higher than that in patients with rheumatoid arthritis or patients with osteoarthritis. When the positive value is set at 67 pmol/L or less, miR-132 can be used to determine that the subject is suffering with each of rheumatoid arthritis and osteoarthritis, at approximately 84% of sensitivity and approximately 80% of specificity. The fact that the AUCs of ROC curves in rheumatoid arthritis and osteoarthritis were high values, 0.90 and 0.91, respectively, shows that the examination method of the present invention is a superior test.
As shown in Figure 3, the concentration of miR-16 in plasma correlated with DAS28, which is currently the most commonly used as disease activity criteria for rheumatoid arthritis. The concentrations of miR-16, miR-132, miR-146a, miR-155, and miR-223 in plasma correlated with TJC (tender joint count).
As shown in Figure 4, the synovial fluid/plasma concentration ratios of miR-16, miR-132, and miR-146a correlated with TJC (tender joint count).

### INDUSTRIAL APPLICABILITY

In the present invention, the contraction or the activity of diseases related immune system and/or joint system can be easily determined by measuring the concentration of miRNA in a blood-derived or joint-derived fluid sample. Therefore, it is considered that the examination of the present invention is carried out as screening, and positive persons are subjected to further examinations, such as an additional blood collection, or a radiography accompanied by radiation exposure.
Although the present invention has been described with reference to specific embodiments, various changes and modifications obvious to those skilled in the art are possible without departing from the scope of the appended claims.

## Claims

1. A method for detecting diseases related to immune system and/or joint system,
wherein at least miRNAs selected from the group consisting of SEQ ID NOS: 1 to 5 contained in a blood-derived or joint-derived fluid sample is used as an index.

2. An examination method for determining the activity of diseases related to immune system and/or joint system, comprising::
preparing blood-derived or joint-derived fluid samples collected over time,
measuring the expression levels of at least an miRNA selected from SEQ ID NOS: 1 to 5 in the fluid samples, and
comparing the expression levels between different sampling times.

3. An examination method for determining the activity of diseases related to immune system and/or joint system, comprising:
preparing blood-derived fluid samples and joint-derived fluid samples collected over time,
measuring the expression levels of at least an miRNA selected from SEQ ID NOS: 1 to 5 in the fluid samples, and
comparing ratios (Ea/Eb) of the expression level (Ea) of miRNA obtained from the joint-derived fluid samples to the expression level (Eb) of miRNA obtained from the blood-derived fluid samples between different sampling times.

4. An examination method for determining the contraction of diseases related to immune system and/or joint system, comprising:
measuring the expression level of miRNA of SEQ ID NO: 2 in a blood-derived fluid sample.

5. An examination method for differentiating diseases related to immune system and/or joint system, comprising:
measuring the expression level of at least an miRNA selected from the group consisting of SEQ ID NOS: 1, 3, 4, and 5 in a joint-derived fluid sample.

6. The method according to any one of claims 1 to 5, wherein the blood-derived fluid sample is plasma, and the joint-derived fluid sample is synovial fluid.

7. Use of a primer or a probe capable of analyzing the expression level of miRNA selected from the group consisting of SEQ ID NOS: 1 to 5 in the manufacture of a kit for detecting diseases related to immune system and/or joint system.


SEQUENCE LISTING

<110> Yoshitomi, Hiroyuki
Murata, Koichi
Mitsubishi Chemical Medience Corporation

<120> Examination method to determine contraction or activity of diseases related immune system or joint system

<130> U2829 EP

<140> EP 11 76 6005.0
<141> 2011-04-08

<150> JP 2010-101467
<151> 2010-04-08

<160> 11

<170> PatentIn version 3.1

<210> 1
<211> 22
<212> RNA
<213> homo sapiens

<400> 1
uagcagcacg uaaauauugg cg 22

<210> 2
<211> 22
<212> RNA
<213> homo sapiens

<400> 2
uaacagucua cagccauggu cg 22

<210> 3
<211> 22
<212> RNA
<213> homo sapiens

<400> 3
ugagaacuga auuccauggg uu 22

<210> 4
<211> 23
<212> RNA
<213> homo sapiens

<400> 4
uuaaugcuaa ucgugauagg ggu 23

<210> 5
<211> 22
<212> RNA
<213> homo sapiens

<400> 5
ugucaguuug ucaaauaccc ca 22

<210>  6
<211>  22
<212>  DNA
<213>  homo sapiens

<400>  6
tagcagcacg taaatattgg cg                                                22


<210>  7
<211>  22
<212>  DNA
<213>  homo sapiens

<400>  7
taacagtcta cagccatggt cg                                                22


<210>  8
<211>  22
<212>  DNA
<213>  homo sapiens

<400>  8
tgagaactga attccatggg tt                                                22


<210>  9
<211>  24
<212>  DNA
<213>  homo sapiens

<400>  9
ttaatgctaa tcgtgatagg ggta                                              24


<210>  10
<211>  22
<212>  DNA
<213>  homo sapiens

<400>  10
tgtcagtttg tcaaataccc ca                                                22


<210>  11
<211>  24
<212>  DNA
<213>  C. elegans

<400>  11
cgtcaccggg tgtaaatcag cttg                                              24